(19) Europäisches Patentamt
European Patent Office
Office européen des brevets

(11) Numéro de publication: **0 031 266**
**B1**

(12) **FASCICULE DE BREVET EUROPEEN**

(45) Date de publication du fascicule du brevet: **09.02.83**

(21) Numéro de dépôt: **80401713.5**

(22) Date de dépôt: **01.12.80**

(51) Int. Cl.³: **C 07 D 333/54,** C 07 D 333/80, C 07 D 333/78, C 07 D 409/12, A 61 K 31/38, A 61 K 31/44

(54) **Nouveaux dérivés de l'amino-1 propanol-2, leur procédé de préparation et leur application thérapeutique.**

(30) Priorité: **21.12.79 FR 7931402**

(43) Date de publication de la demande: **01.07.81 Bulletin 81/26**

(45) Mention de la délivrance du brevet: **09.02.83 Bulletin 83/6**

(84) Etats contractants désignés: **DE NL**

(56) Documents cités:
**FR-M-6 997**
**FR-M-8 254**

(73) Titulaire: **SANOFI, Société dite:, 40, Avenue George V, F-75008 Paris (FR)**

(72) Inventeur: **Courregelongue, Jean, 3, Rue Georges Clémenceau, F-31120 Portet/Garonne (FR)**
Inventeur: **Maffrand, Jean-Pierre, 5, Rue du Corps-Franc Pommiès, F-31120 Portet/Garonne (FR)**

(74) Mandataire: **Lavoix, Jean et al, c/o Cabinet Lavoix 2, Place D'Estienne D'Orves, F-75441 Paris Cedex 09 (FR)**

**EP 0 031 266 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Nouveaux dérivés de l'amino-1 propanol-2, leur procédé de préparation et leur application thérapeutique

La présente invention est relative à de nouveaux dérivés de l'amino-1 propanol-2, à leur procédé de préparation et à leur application en médecine humaine et vétérinaire.

Les nouveaux composés de l'invention répondent aux formules générales suivantes:

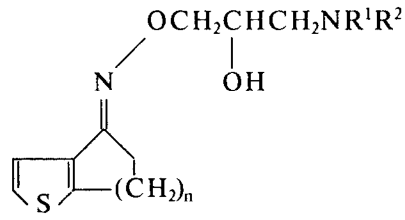

isomères E (I)

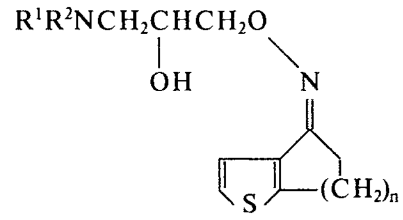

isomères Z (II)

dans lesquelles $R^1$ et $R^2$ représentent indépendamment l'un de l'autre un atome d'hydrogène; un groupe alcoyle linéaire ou ramifié ayant de 1 à 10 atomes de carbone; cycloalcoyle mono- ou polycyclique ayant de 1 à 14 atomes de carbone, éventuellement mono- ou polysubstitué par un groupe alcoyle ayant de 1 à 4 atomes de carbone; alcényle linéaire ou ramifié ayant de 3 à 7 atomes de carbone; alcynyle linéaire ou ramifié ayant de 3 à 7 atomes de carbone; phinyle ou phénylalcoyle, ayant de 1 à 4 atomes de carbone dans la fraction alcoyle, éventuellement mono- ou polysubstitué sur le noyau aromatique par un atome d'halogène, un groupe hydroxy, alcoyle en $C_1$ à $C_4$ ou alcoxy en $C_1$ à $C_4$; pyridile ou pyridylaralcoyle ayant de 1 à 4 atomes de carbone dans la fraction alcoyle; aminoalcoyle en $C_1$ à $C_4$, éventuellement mono- ou disubstitué sur l'atome d'azote par un groupe alcoyle en $C_1$ à $C_4$; ou bien $R^1$ et $R^2$ forment avec l'atome d'azote sur lequel ils sont fixés un hétérocycle ayant de 5 à 7 chaînons, contenant éventuellement un autre hétéroatome choisi parmi l'oxygène, le soufre et l'azote, ce dernier étant éventuellement substitué par un groupe alcoyle en $C_1$ à $C_6$, phényle, phénylalcoyle ou benzoyle dans lesquels le noyau aromatique est éventuellement mono- ou polysubstitué par un atome d'halogène, un groupe hydroxy, alcoyle en $C_1$ à $C_4$ ou alcoxy en $C_1$ à $C_4$; et n est un nombre entier de 1 à 3, ainsi que leurs sels d'addition avec des acides minéraux ou organiques pharmaceutiquement acceptables.

Dans la définition donnée ci-dessus, on désigne sous la définition phénylalcoyle un groupe tel que benzyle, phénéthyle ou benzhydryle.

Sous la désignation hétérocycle saturé, on vise plus spécialement des groupes tels que pyrrolidino, pipérazino, pipéridino, morpholino.

Les groupes alcényles linéaires ou ramifiés sont notamment un groupe $\alpha,\alpha$-dialcoylallyle et les groupes alcynyle linéaires ou ramifiés, un groupe $\alpha,\alpha$-dialcoylpropynyle.

Les composés de formule (I) et (II) possèdent un atome de carbone asymétrique et peuvent, par conséquent, exister sous forme d'isomères optiques. Ces formes peuvent être obtenues par des méthodes classiques et l'invention concerne aussi bien les stéréoisomères purs que leur mélange.

L'invention vise par ailleurs un procédé de préparation des composés de formule (I) et (II) ainsi définis.

Ce procédé consiste:

a) à condenser des oximes de formules (III) ou (IV) suivantes:

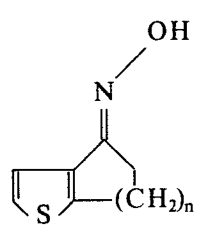

(III)

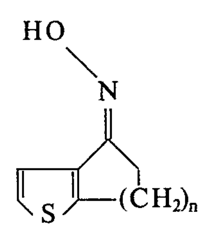

(IV)

avec une épihalohydrine, dans un solvant inerte, en présence d'une base, à une température comprise entre 10° C et le point d'ébullition du solvant, pour obtenir des composés de formules:

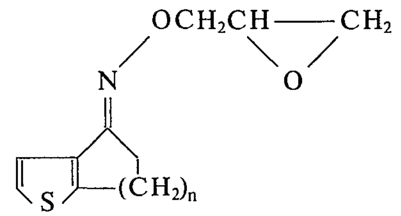 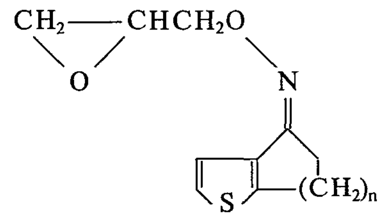

(V) (VI)

dans lesquelles n a les significations données précédemment.

b) à condenser les composés de formules (V) et (VI) obtenus avec une amine de formule $HNR^1R^2$ dans laquelle $R^1$ et $R^2$ sont tels que définis ci-dessus, dans un solvant inerte, à une température comprise entre 10° C et la température d'ébullition du solvant pour obtenir respectivement les dérivés de formule (I) ou (II).

La réaction de condensation de l'étape a) est réalisée en utilisant de préférence l'épichlorhydrine dans un solvant inerte choisi parmi le diméthylformamide, le méthanol ou l'éthanol et en présence comme base, notamment, d'un carbonate de métal alcalin tel que le carbonate de potassium.

Dans la réaction de condensation de l'étape b), on utilise notamment comme solvant inerte un alcanol inférieur tel que l'éthanol ou le méthanol et la réaction est avantageusement mise en oeuvre avec un excès d'amine.

Le dérivé de formule (III) dans lequel n = 2 est déjà décrit, M. C. KLOETZEL, J. E. LITTLE, Jr, et D. M. FRISCH, J. Org. Chem., 1953, 18, 1511. Mais sa préparation s'accompagne toujours de celle d'une certaine quantité de son isomère (IV) (n = 2) qui a pu être isolé par chromatographie sur colonne de silice (éluant : toluène-acétate d'éthyle 9/1). F = 140° C (éther isopropylique).

L'oxime de formule (III) dans laquelle n = 3 est également connue: P. CAGNIANT et D. CAGNIANT; Bull. Soc. Chim. France, 1955, 680. Son isomère de formule IV qui n'était pas évoqué dans l'article indiqué précédemment a été obtenu en faible quantité dans le milieu réactionnel et a pu être isolé par chromatographie sur colonne de silice (éluant : toluène) F = 68° C.

L'oxime de formule (III) dans laquelle n = 1 a été préparée à partir de la cétone correspondante décrite dans la littérature: J. SAM, A. C. THOMPSON, J. Pharm. Sci., 1963, 52, 898. Son isomère de formule (IV) dans laquelle n = 1 n'a pas pu être mis en évidence dans le milieu réactionnel.

Les exemples non limitatifs suivants illustrent l'invention:

## Exemple1

E-(tert-butylamino-3 hydroxy-2 propoxy) imino-4 tétrahydro-4,5,6,7 benzo (b) thiophène (I)
($R^1$ = H, $R^2$ = tBu; n = 2)

### a) Préparation des isomères E ou Z de l'(époxy-2,3 propoxy)imino-4 tétrahydro-4,5,6,7 benzo (b) thiophène

On chauffe à 110°C pendant 6 heures un mélange de 100 g (0,60 mole) d'oxime E de l'oxo-4 tétrahydro-4,5,6,7 benzo (b) thiophène, 330 g (3,6 moles) d'épichlorhydrine et 391,2 g (2,8 moles) de carbonate de potassium dans un litre de diméthylformamide.

Après filtration des sels minéraux et évaporation à sec du filtrat, l'huile brune obtenue est dissoute dans de l'acétate d'éthyle. La phase organique est lavée à l'eau, séchée sur sulfate de sodium et évaporée à sec. L'huile résiduelle est chromatographiée sur une colonne de silice (éluant : toluène-acétate d'éthyle 95/5). On récupère l'isomère E sous forme d'huile orangée (rendement: 83%). De la même façon à partir de l'oxime Z isolée on prépare l'isomère Z; huile verdâtre, rendement: 56%.

### b) Préparation du dérivé 1

On chauffe à reflux pendant 4 heures une solution de 8 g (0,036 mole) de E-(époxy-2,3 propoxy)imino-4 tétrahydro-4,5,6,7 benzo (b) thiophène et 5,3 g (2 équivalents) de tertiobutylamine dans 100 cm³ d'éthanol.

On évapore à sec et chromatographie l'huile résiduelle sur une colonne de silice (éluant : chlorure de méthylène-méthanol 9/1). Le produit récupéré est transformé en maléate: cristaux blancs, F = 148° C (isopropanol-éther isopropylique), rendement: 55%.

### Exemple 2

E-(hydroxy-2 isopropylamino-3 propoxy) imino-4 tétrahydro-4,5,6,7 benzo (b) thiophène (I)
($R^1 = H$, $R^2 = iPr$; $n = 2$)

Ce composé est préparé selon le mode opératoire décrit dans l'exemple 1 par condensation du E-(époxy-2,3 propoxy) imino-4 tétrahydro-4,5,6,7 benzo (b) thiophène et de l'isopropylamine. Oxalate: cristaux blancs, F = 150° C (isopropanol-éther isopropylique), rendement: 62,5%.

### Exemple 3

E-(cyclohexylamino-3 hydroxy-2 propoxy) imino-4 tétrahydro-4,5,6,7 benzo (b) thiophène (I)
($R^1 = H$, $R^2 =$ cyclohexyl; $n = 2$)

Ce composé est préparé selon le mode opératoire décrit dans l'exemple 1 par condensation du E-(époxy-2,3 propoxy) imino-4 tétrahydro-4,5,6,7 benzo (b) thiophène et de la cyclohexylamine. Maléate: cristaux blancs, F = 138° (isopropanol), rendement 77%.

### Exemple 4

E-[(adamantyl-1)amino-3 hydroxy-2 propoxy] imino-4 tétrahydro-4,5,6,7 benzo (b) thiophène (I)
($R^1 = H$, $R^2 =$ adamantyl; $n = 2$)

Ce composé est préparé selon le mode opératoire décrit dans l'exemple 1 par condensation du E-(époxy-2,3 propoxy)imino-4 tétrahydro-4,5,6,7 benzo (b) thiophène et de l'adamantane-1 amine. Maléate: cristaux blancs, F = 204° C (éthanol), rendement 56%.

### Exemple 5

E-(cyclododécylamino-3 hydroxy-2 propoxy)imino-4 tétrahydro-4,5,6,7 benzo (b) thiophène (I) ($R^1 = H$, $R^2 =$ cyclododécyl; $n = 2$)

Ce composé est préparé selon le mode opératoire décrit dans l'exemple 1 par condensation du E-(époxy-2,3 propoxy)imino-4 tétrahydro-4,5,6,7 benzo (b) thiophène et de la cyclododécylamine. Maléate: cristaux blancs, F = 164° C (isopropanol), rendement 70%.

### Exemple 6

E-[(diéthyl-1,1 propyne-2 yl) amino-3 hydroxy-2 propoxy] imino-4 tétrahydro-4,5,6,7 benzo (b) thiophène (I)
($R^1 = H$, $R^2 = -C(C_2H_5)_2C{\equiv}CH$; $n = 2$)

Ce composé est préparé selon le mode opératoire dècrit dans l'exemple 1 par condensation du E-(époxy-2,3 propoxy) imino-4 tétrahydro-4,5,6,7 benzo (b) thiophène et de la diéthyl-2,2 propargylamine.

Oxalate: cristaux blancs, F = 150° C (isopropanol), rendement: 81,5%.

### Exemple 7

E-[hydroxy-2(méthyl-4 pipérazinyl-1)-3 propoxy] imino-4 tétrahydro-4,5,6,7 benzo (b) thiophène (I)

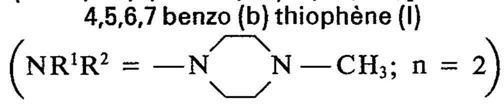

Ce composé est préparé selon le mode opératoire décrit dans l'exemple 1 par condensation du E-(époxy-2,3 propoxy)imino-4 tétrahydro-4,5,6,7 benzo (b) thiophène et de la méthyl-1 pipérazine. Dioxalate: cristaux blancs, F = 198° C (méthanol-eau), rendement: 66%.

Exemple 8

E-[hydroxy-2(pyrrolidinyl-1)-3 propoxy] imino-4 tétrahydro-4,5,6,7 benzo (b) thiophène (I)

($NR^1R^2$ = 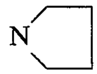 ; n = 2)

Ce composé est préparé selon le mode opératoire décrit dans l'exemple 1 par condensation du E-(époxy-2,3 propoxy)imino-4 tétrahydro-4,5,6,7 benzo (b) thiophène et de la pyrrolidine. Oxalate: cristaux blancs, F = 136° C (isopropanol), rendement 77,5%.

Exemple 9

E-(hydroxy-2 morpholino-3 propoxy) imino-4 tétrahydro-4,5,6,7 benzo (b) thiophène (I)

($NR^1R^2$ = 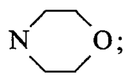; n = 2)

Ce composé est préparé selon le mode opératoire décrit dans l'exemple 1 par condensation du E-(époxy-2,3 propoxy) imino-4 tétrahydro-4,5,6,7 benzo (b) thiophène et de la morpholine.
Oxalate: cristaux blancs, F = 152° C (isopropanol-méthanol), rendement 76%.

Exemple 10

E-[hydroxy-2 (diméthoxy-3,4 phénéthylamino)-3 propoxy] imino-4 tétrahydro-4,5,6,7 benzo (b) thiophène (I)
($R^1 = H$, $R^2 = 3{,}4\text{-di-MeO-}C_6H_3\text{-}(CH_2)_2$; n = 2)

Ce composé est préparé selon le mode opératoire décrit dans l'exemple 1 par condensation de la diméthoxy-3,4 phénéthylamine et du E-(époxy-2,3 propoxy) imino-4 tétrahydro-4,5,6,7 benzo (b) thiophène.
Oxalate: cristaux blancs, F = 167° C (éthanol), rendement: 48%. Chlorhydrate: cristaux blancs, F = 134° C (isopropanol), rendement: 23,5%.

Exemple 11

E-(benzylamino-3 hydroxy-2 propoxy) imino-4 tétrahydro-4,5,6,7 benzo (b) thiophène (I)
($R^1 = H$, $R^2 = C_6H_5\text{-}CH_2$; n = 2)

Ce composé est préparé selon le mode opératoire décrit dans l'exemple 1 par condensation du E-(époxy-2,3 propoxy) imino-4 tétrahydro-4,5,6,7 benzo (b) thiophène et de la benzylamine.
Oxalate: cristaux blancs, F = 207° C (isopropanol), rendement 36%.

Exemple 12

E-(hydroxy-2 pipéridino-3 propoxy) imino-4 tétrahydro-4,5,6,7 benzo (b) thiophène (I)

($NR^1R^2$ = 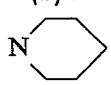 ; n = 2)

Ce composé est préparé selon le mode opératoire décrit dans l'exemple 1 par condensation du E-(époxy-2,3 propoxy) imino-4 tétrahydro-4,5,6,7 benzo (b) thiophène et de la pipéridine.
Hémifumarate: cristaux blancs, F = 146° C (isopropanol), rendement: 66%.

### Exemple 13

E-[(benzyl-4 pipérazinyl-1)-3 hydroxy-2 propoxy] imino-4 tétrahydro-4,5,6,7 benzo (b) thiophène (I)

($NR^1R^2$ = 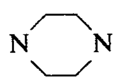 $-CH_2-C_6H_5$; n = 2)

Ce composé est préparé selon le mode opératoire décrit dans l'exemple 1 par condensation du E-(époxy-2,3 propoxy) imino-4 tétrahydro-4,5,6,7 benzo (b) thiophène et de la benzyl-1 pipérazine. Dimaléate: cristaux blancs, F = 210° C (méthanol-eau), rendement: 46%.

### Exemple 14

E-(hydroxy-2 octylamino-3 propoxy) imino-4 tétrahydro-4,5,6,7 benzo (b) thiophène (I)
($R^1$ = H, $R^2$ = octyle; n = 2)

Ce composé est préparé selon le mode opératoire décrit dans l'exemple 1 par condensation du E-(époxy-2,3 propoxy) imino-4 tétrahydro-4,5,6,7 benzo (b) thiophène et de l'octylamine. Oxalate: cristaux blancs, F = 152° C (isopropanol), rendement: 43%.

### Exemple 15

E-[hydroxy-2 (pyridyl-3 méthyl) amino-3 propoxy] imino-4 tétrahydro-4,5,6,7 benzo (b) thiophène (I)

($R^1$ = H, $R^2$ = $-CH_2-$ 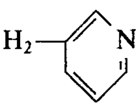 ; n = 2)

Ce composé est préparé selon le mode opératoire décrit dans l'exemple 1 par condensation du E-(époxy-2,3 propoxy) imino-4 tétrahydro-4,5,6,7 benzo (b) thiophène et de la picolylamine. Dioxalate: cristaux beiges, F = 214° C (méthanol), rendement 30%.

### Exemple 16

E-(cyclopentylamino-3 hydroxy-2 propoxy) imino-4 tétrahydro-4,5,6,7 benzo (b) thiophène (I)
($R^1$ = H, $R^2$ = cyclopentyl; n = 2)

Ce composé est préparé selon le mode opératoire décrit dans l'exemple 1 par condensation du E-(époxy-2,3 propoxy) imino-4 tétrahydro-4,5,6,7 benzo (b) thiophène et de la cyclopentylamine. Maléate: cristaux blancs, F = 120° C (isopropanol), rendement: 64%.

### Exemple 17

E-[diméthyl-1,1 propyne-2 yl)amino-3 hydroxy-2 propoxy] imino-4 tétrahydro-4,5,6,7 benzo (b) thiophène (I)
($R^1$ = H, $R^2$ = $C(CH_3)_2C\equiv CH$; n = 2)

Ce composé est préparé selon le mode opératoire décrit dans l'exemple 1 par condensation du E-(époxy-2,3 propoxy) imino-4 tétrahydro-4,5,6,7 benzo (b) thiophène et de la diméthyl-2,2 propargylamine. Hémioxalate: cristaux blancs, F = 204° C (méthanol-eau), rendement: 44%.

Exemple 18

E-(hydroxy-2β-diméthylamino éthylamino-3 propoxy) imino-4 tétrahydro-4,5,6,7 benzo (b) thiophène (I)
($R^1 = H$, $R^2 = (CH_3)_2N-(CH_2)_2$; $n = 2$)

Ce composé est préparé selon le mode opératoire décrit dans l'exemple 1 par condensation du E-(époxy-2,3 propoxy) imino-4 tétrahydro-4,5,6,7 benzo (b) thiophène et de la β-diméthylamino-éthylamine.
Dioxalate: cristaux blancs, F = 222° C (méthanol-eau), rendement: 28%.

Exemple 19

E-(hydroxy-2 isopropylamino-3 propoxy) imino-4 tétrahydro-5,6,7,8 4H-cyclohepta(b)thiophène (I)
($R^1 = H$, $R^2 = iPr$; $n = 3$)

Ce composé est préparé selon le mode opératoire décrit dans l'exemple 1 par condensation du E-(époxy-2,3 propoxy) imino-4 tétrahydro-5,6,7,8 4H-cyclohepta (b) thiophène et de l'isopropylamine.
Hémioxalate: cristaux blancs, F = 176° C (acétone-éthanol), rendement: 18%.

Exemple 20

E-(hydroxy-2 isopropylamino-3 propoxy) imino-4 dihydro-5,6, 4H cyclopenta (b) thiophène (I)
($R^1 = H$, $R^2 = iPr$; $n = 1$)

Ce composé est préparé selon le mode opératoire décrit dans l'exemple 1 par condensation du E-(époxy-2,3 propoxy) imino-4 dihydro-5,6 4H-cyclopenta (b) thiophène et de l'isopropylamine.
Hémioxalate: cristaux blancs, F = 216° C (méthanol), rendement: 61%.

Exemple 21

Z-(hydroxy-2 isopropylamino-3 propoxy) imino-4 tétrahydro-4,5,6,7 benzo (b) thiophène (II)
($R^1 = H$, $R^2 = iPr$; $n = 2$)

Ce composé est préparé selon le mode opératoire décrit dans l'exemple 1 par condensation du Z-(époxy-2,3 propoxy) imino-4 tétrahydro-4,5,6,7 benzo (b) thiophène et de l'isopropylamine.
Oxalate: cristaux blancs, F = 138° C (isopropanol), rendement: 20%.

Les résultats des essais toxicologiques et pharmacologiques qui sont rapportés ci-après mettent en évidence la faible toxicité et la bonne tolérance des dérivés de formule I ou II, ainsi que leurs activités, notamment inhibitrice sur les récepteurs β-adrénergiques et antiarythmique.

La présente invention a donc également pour objet un médicament, caractérisé en ce qu'il contient, à titre de principe actif, une quantité efficace d'un dérivé de formule I et/ou II ou un sel d'addition avec un acide pharmaceutiquement acceptable de ce dernier.

I — Etude toxicologique

Les dérivés de formule I ou II bénéficient d'une excellente tolérance et d'une faible toxicité. Ainsi, la DL 50/24h/kg de poids corporel déterminée chez la souris selon la méthode de Miller et Tainter par la voie intraveineuse est de 28,4 mg pour le dérivé n° 1, de 39,2 mg pour la dérivé n° 2, de 40,5 mg pour le dérivé n° 6, de 123,4 mg pour le dérivé n° 7, de 32,1 mg pour le dérivé n° 8, de 74,9 mg pour le dérivé n° 9, de 49,5 mg pour le dérivé n° 10, de 22,2 mg pour le dérivé n° 12, de 99,3 mg pour le dérivé n° 15, de 32,4 mg pour le dérivé n° 16 et de 84,4 mg pour le dérivé n° 18.

En outre, les essais effectués sur la toxicité aigüe, chronique, subchronique et retardée chez différentes espèces animales, n'ont mis en évidence aucune réaction locale ou générale, aucune perturbation dans les contrôles biologiques régulièrement effectués, aucune anomalie dans les examens microscopiques et macroscopiques pratiqués sur les animaux sacrifiés et autopsiés en fin d'expérimentation. Les dérivés de formule I ou II sont en outre dépourvus d'effet tératogène.

## II — Etude pharmacologique

### 1°) Action inhibitrice sur les récepteurs β-adrénergiques

#### a) In vitro

Cette étude a été effectuée sur l'oreillette isolée de cobaye battant spontanément d'après la méthode de K. SAAMELI, Helv. Physiol. Acta 25, 219—221, 1967.

Les oreillettes droite et gauche sont placées dans une solution de Tyrode modifiée et oxygénée à 30° C, et on enregistre en continu les variations de l'amplitude des contractions. Après 30 minutes, on ajoute dans le milieu 0,5 ml d'une solution de chlorhydrate de 1-adrénaline contenant 2,5 μmoles/ml d'adrénaline et on laisse en contact une minute. On lave les oreillettes avec la solution de Tyrode et 20 minutes plus tard, on ajoute la substance à tester à une concentration déterminée; 20 minutes plus tard, on ajoute de nouveau 0,5 ml de la solution d'adrénaline. On détermine ainsi:

- l'action des composés testés sur l'amplitude des contractions, c'est-à-dire leur activité inotrope négative;
- l'action inhibitrice des composés testés vis-à-vis de l'adrénaline au niveau des récepteurs β-adrénergiques.

Ajoutés à diverses concentrations dans le milieu où baignent les oreillettes de cobaye isolées et battant spontanément, les composés de l'invention vont exercer un effet antagoniste net envers l'action inotrope positive produite par l'adrénaline sur la fréquence et l'amplitude des contractions. Ainsi, par exemple, pour le composé n° 1, on a déterminé que, introduit dans le milieu, à la concentration de 0,050 mg/litre, il réduit de plus de 15% l'amplitude des contractions mesurée avant l'introduction dans le milieu de l'adrénaline.

En outre, à une concentration de 0,05 mg/litre, ce composé inhibe à plus de 50% l'effet inotrope positif de l'adrénaline, c'est-à-dire qu'il réduit de plus de 50% l'amplitude des contractions produites par l'adrénaline seule.

#### b) In vivo

L'expérimentation a été effectuée sur le chien. L'animal, anesthésié par une injection intraveineuse de pentobarbital, reçoit par la voie intraveineuse, 0,1 μg/kg d'isoprénaline. Deux minutes après l'injection, on mesure la pression artérielle, la fréquence cardiaque et la force contractile du muscle cardiaque, et on injecte dans la veine fémorale le produit à tester. Les différents paramètres sont mesurés de nouveau et on constate que les composés testés inhibent les désordres provoqués par l'isoprénaline et ramènent à la normale l'activité cardiaque perturbée.

Les résultats rassemblés ci-après indiquent les doses des composés les plus actifs produisant une inhibition totale des effets de l'isoprénaline.

| Dérivé testé | Dose efficace en mg/kg |
|---|---|
| 1 | 2,50 |
| 2 | 2,9 |
| 3 | 2,20 |
| 4 | 2,50 |
| 6 | 3,0 |
| 7 | 2,80 |
| 8 | 2,1 |
| 9 | 2,50 |
| 10 | 3,0 |
| 12 | 2,3 |
| 13 | 2,2 |
| 15 | 2,8 |
| 16 | 2,4 |
| 18 | 2,5 |

2°) Action antiarythmique

Cette activité a été étudiée chez le chien: le test consiste en l'occlusion par ligature en un temps de l'artère coronaire interventriculaire antérieure (HARRIS, A. S., CIRCULATION, 1950, 1 [6], 1318). L'anoxie qui en résulte entraîne des modifications électrophysiologiques de la cellule myocardiaque provoquant une tachycardie ventriculaire ou une arythmie polymorphe.

Les troubles débutent environ 4 heures après la ligature, l'acmée étant atteinte de 10 à 20 heures après l'intervention. Le retour à la normale se produit, en général, au bout de 72 heures.

Les substances antiarythmiques à tester sont administrées pendant la periode de troubles maxima à la dose de 10 mg/kg; on constate que les composés de l'invention font rapidement disparaître les troubles du rythme artificiellement provoqués.

Le médicament de l'invention peut être présenté pour l'administration orale, sous forme de comprimés, comprimés dragéifiés, capsules, gouttes ou sirop.

Il peut aussi être présenté pour l'administration rectale sous forme de suppositoires et pour l'administration parentérale sous forme de soluté injectable. Chaque dose unitaire contient avantageusement de 0,005 g à 0,100 g de principe actif associé à divers excipients pharmaceutiquement compatibles et les doses journalières pourront varier de 0,005 g à 0,300 g en fonction de l'âge du patient et de la gravité de l'affection traitée.

On donnera, ci-après, à titre d'exemple non limitatif, quelques formulations pharmaceutiques du médicament de l'invention.

1/Comprimés
dérivé 2 — 0,010 g
excipient: amidon, talc, glucose, stéarate de magnésium.

2/Comprimés dragéifiés
dérivé 4 — 0,025 g
excipient: lactose, amidon de maïs, polyvinylpyrrolidone, stéarate de magnésium, talc, gomme arabique, gomme laque, huile de ricin, oxyde de titane, cire blanche, cire de carnauba.

3/Capsules
dérivé 7 — 0,020 g
excipient: aérosil, talc, stéarate de magnesium.

4/Suppositoires
dérivé 12 — 0,050 g
excipient: triglycérides semi-synthétiques.

5/Soluté injectable
dérivé 20 — 0,025 g
excipient: soluté isotonique q.s.p. — 5 ml

Grâce à ses propriétés inhibitrice sur les récepteurs $\beta$-adrénergiques et anti-arythmique, le médicament de l'invention peut être utilisé avantageusement en thérapeutique humaine. Il est administré avec profit dans le traitement des troubles du rythme et de l'hypertension artérielle.

**Revendications**

1. Dérivés de l'amino-1 propanol-2 répondant aux formules suivantes:

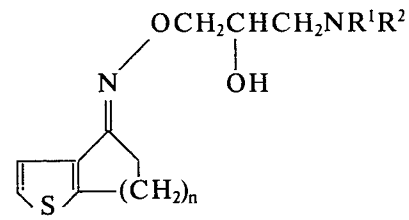

isomères E (I)

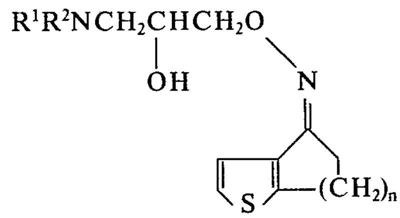

isomères Z (II)

dans lesquelles $R^1$ et $R^2$ représentent indépendamment l'un de l'autre un atome d'hydrogène; un groupe alcoyle linéaire ou ramifié ayant de 1 à 10 atomes de carbone; cycloalcoyle mono- ou polycyclique ayant de 1 à 14 atomes de carbone, éventuellement mono- ou polysubstitué par un groupe alcoyle ayant de 1 à 4 atomes de carbone; alcényle linéaire ou ramifié ayant de 3 à 7 atomes de carbone; alcynyle linéaire ou ramifié ayant de 3 à 7 atomes de carbone; phényle ou phénylalcoyle ayant de 1 à 4 atomes de carbone dans la fraction alcoyle, éventuellement mono- ou polysubstitué sur le noyau aromatique par un atome d'halogène, un groupe hydroxy, alcoyle en $C_1$ à $C_4$ ou alcoxy en $C_1$ à $C_4$; pyridyle ou pyridylalcoyle ayant de 1 à 4 atomes de carbone dans la fraction alcoyle; aminoalcoyle en $C_1$ à $C_4$, éventuellement mono- ou disubstitué sur l'atome d'azote par un groupe alcoyle en $C_1$ à $C_4$;
ou bien $R^1$ et $R^2$ forment avec l'atome d'azote sur lequel ils sont fixés un hétérocycle ayant de 5 à 7 chaînons, contenant éventuellement un autre hétéroatome choisi parmi l'oxygène, le soufre et l'azote, ce dernier étant éventuellement substitué par un groupe alcoyle en $C_1$ à $C_6$, phényle, phénylalcoyle dans lesquels le noyau aromatique est éventuellement mono- ou polysubstitué par un atome d'halogène, un groupe hydroxy, alcoyle en $C_1$ à $C_4$ ou alcoxy en $C_1$ à $C_4$; et n est un nombre entier de 1 à 3, ainsi que leurs sels d'addition avec des acides minéraux ou organiques pharmaceutiquement acceptables.

2. Dérivés selon la revendication 1, répondant aux formules I et II, dans lesquelles $R^1$ et $R^2$ représentent indépendamment l'un de l'autre un atome d'hydrogène; un groupe alcoyle linéaire ou ramifié ayant de 1 à 8 atomes de carbone; cycloalcoyle monocyclique ayant de 1 à 12 atomes de carbone, ou adamantyle; $\alpha,\alpha$-dialcoyle ($C_{1-4}$) propynyle; phénylalcoyle ($C_{1-4}$), éventuellement mono- ou polysubstitué sur le noyau phényle par un groupe alcoxy en $C_1$ à $C_4$; pyridylalcoyle ($C_{1-4}$); dialcoyl ($C_{1-4}$) aminoalcoyle ($C_{1-4}$);
ou bien $R^1$ et $R^2$ forment avec l'atome d'azote sur lequel ils sont fixés un groupe pyrrolidino, morpholino, pipéridino ou pipérazino, ce dernier étant éventuellement substitué sur un autre atome d'azote par un groupe alcoyle en $C_1$ à $C_4$, ou benzyle, et n est un nombre entier de 1 à 3, ainsi que leurs sels d'addition avec des acides minéraux ou organiques pharmaceutiquement acceptables.

3. Procédé de préparation de dérivés de l'amino-1 propanol-2 selon la revendication 1, caractérisé en ce qu'il consiste:

a) à condenser des oximes de formules (III) ou (IV) suivantes:

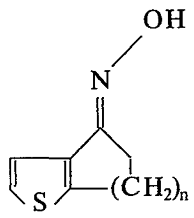

(III)

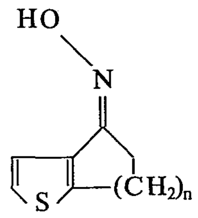

(IV)

avec une épihalohydrine, dans un solvant inerte, en présence d'une base, à une température comprise entre 10° C et le point d'ébullition du solvant, pour obtenir des composés de formules:

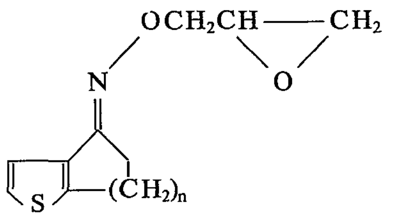

(V)

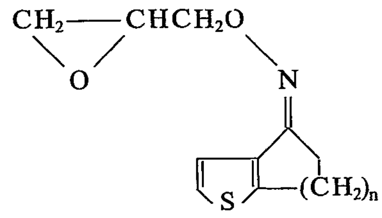

(VI)

dans lesquelles n a les significations données précédemment;

b) à condenser les composés de formule (V) et (VI) obtenus avec une amine de formule $HNR^1R^2$ dans laquelle $R^1$ et $R^2$ sont tels que définis ci-dessus, dans un solvant inerte, à une température comprise entre 10° C et la température d'ébullition du solvant pour obtenir respectivement les dérivés de formule (I) ou (II).

4. Procédé selon la revendication 3, caractérisé en ce que la réaction de condensation de l'étape a) est réalisée en utilisant l'épichlorhydrine dans un solvant inerte choisi parmi le diméthylformamide, le méthanol et l'éthanol et en présence d'un carbonate de métal alcalin comme base.

5. Procédé selon la revendication 3, caractérisé en ce que la réaction de condensation de l'étape b) est avantageusement réalisée en présence d'un excès de l'amine, dans un alcanol inférieur comme solvant inerte.

6. Médicament ayant notamment des activités inhibitri_e des récepteurs $\beta$-adrénergiques et antiarythmique, caractérisé en ce qu'il contient à titre de principe actif un composé tel que défini à la revendication 1.

7. Médicament selon la revendication 6, caractérisé en ce qu'il est présenté sous une forme appropriée à l'administration orale, parentérale ou rectale, le principe actif étant associé à des véhicules ou excipients thérapeutiquement acceptables.

8. Médicament selon la revendication 6, caractérisé en ce qu'il est présenté sous forme de doses unitaires contenant chacune de 0,005 à 0,100 g de principe actif.

**Patentansprüche**

1. Derivate von 1-Amino-2-propanol entsprechend den folgenden Formeln:

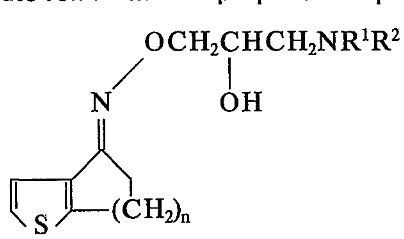

Isomere E (I)

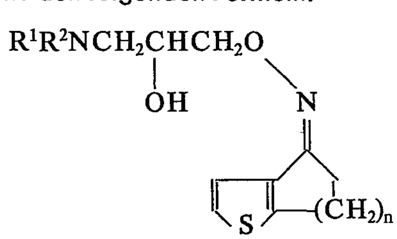

Isomere Z (II)

in denen $R^1$ und $R^2$ unabhängig voneinander ein Wasserstoffatom, eine lineare oder verzweigte Alkylgruppe mit 1 bis 10 C-Atomen, ein gegebenenfalls mit einem Alkylrest mit 1 bis 4 C-Atomen einfach oder mehrfach substituierter mono- oder polycyclischer Cycloalkylrest, ein linearer oder verzweigter Alkenylrest mit 3 bis 7 C-Atomen, ein linearer oder verzweigter Alkinylrest mit 3 bis 7 C-Atomen, ein gegebenenfalls am aromatischen Ring durch ein Halogenatom, eine Hydroxylgruppe,

einen $C_1-C_4$-Alkylrest oder einen $C_1-C_4$-Alkoxyrest einfach oder mehrfach substituierter Phenyl- oder Phenylalkylrest mit 1 bis 4 C-Atomen im Alkylteil, eine Pyridyl- oder Pyridylalkylgruppe mit 1 bis 4 C-Atomen im Alkylteil, ein gegebenenfalls am Stickstoffatom durch einen $C_1-C_4$-Alkylrest einfach oder zweifach substituierter $C_1-C_4$-Aminoalkylrest sind, oder worin $R^1$ und $R^2$ mit dem Stickstoffatom, an das sie gebunden sind, einen Heterocyclus mit 5 bis 7 Ketten bilden, der gegebenenfalls ein aus Sauerstoff, Schwefel und Stickstoff ausgewähltes weiteres Heteroatom enthält, das gegebenenfalls durch einen $C_1-C_6$-Alkylrest, einen Phenylrest, einen Phenylalkylrest, in denen der aromatische Ring gegebenenfalls durch ein Halogenatom, eine Hydroxylgruppe, einen $C_1-C_4$-Alkylrest oder $C_1-C_4$-Alkoxyrest einfach oder mehrfach substituiert ist, substituiert ist, n eine ganze Zahl von 1 bis 3 ist, sowie ihre Additionssalze mit pharmazeutisch unbedenklichen anorganischen oder organischen Säuren.

2. Derivate nach Anspruch 1 entsprechend den Formeln I und II, in denen $R^1$ und $R^2$ unabhängig voneinander ein Wasserstoffatom, eine lineare oder verzweigte Alkylgruppe mit 1 bis 8 C-Atomen, ein monocyclischer Cycloalkylrest mit 1 bis 12 C-Atomen oder Adamantyl, $\alpha,\alpha$-Dialkyl($C_1-C_4$)-propinyl, gegebenenfalls am Phenylring durch einen $C_1-C_4$-Alkoxyrest einfach oder mehrfach substituiertes Phenylalkyl ($C_1-C_4$), Pyridylalkyl ($C_1-C_4$), Dialkyl($C_1-C_4$)-aminoalkyl($C_1-C_4$) sind, oder worin $R^1$ und $R^2$ mit dem Stickstoffatom, an das sie gebunden sind, eine Pyrrolidino-, Morpholino-, Piperidino- oder Piperazinogruppe, wobei die letztere gegebenenfalls an einem anderen Stickstoffatom durch einen $C_1-C_4$-Alkylrest substituiert ist, oder Benzyl bilden, und n eine ganze Zahl von 1 bis 3 ist, sowie ihre Additionssalze mit pharmazeutisch unbedenklichen anorganischen oder organischen Säuren.

3. Verfahren zur Herstellung von 1-Amino-2-propanolderivaten nach Anspruch 1, dadurch gekennzeichnet, daß man

a) Oxime der folgenden Formeln (III) oder (IV)

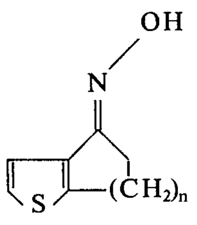
(III)

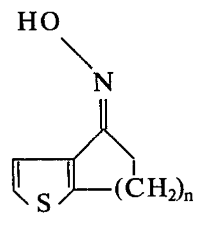
(IV)

mit einem Epihalogenhydrin in einem inerten Lösungsmittel in Gegenwart einer Base bei einer Temperatur zwischen 10°C und dem Siedepunkt des Lösungsmittels kondensiert und hierbei Verbindungen der Formeln

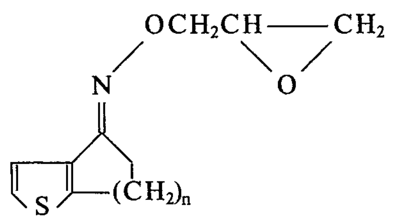
(V)

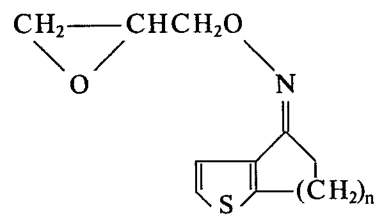
(VI)

erhält, in denen n die vorstehend angegebenen Bedeutungen hat,

b) die erhaltenen Verbindungen der Formeln (V) und (VI) mit einem Amin der Formel $HNR^1R^2$, in der $R^1$ und $R^2$ die vorstehend genannten Bedeutungen haben, in einem inerten Lösungsmittel bei einer Temperatur zwischen 10°C und der Siedetemperatur des Lösungsmittels kondensiert und jeweils die Derivate der Formel (I) oder (II) erhält.

4. Verfahren nach Anspruch 3, dadurch gekennzeichnet, daß die Kondensationsreaktion der Stufe (a) unter Verwendung von Epichlorhydrin in einem aus Dimethylformamid, Methanol und Äthanol ausgewählten inerten Lösungsmittel und in Gegenwart eines Alkalicarbonats als Base durchgeführt wird.

5. Verfahren nach Anspruch 3, dadurch gekennzeichnet, daß die Kondensationsreaktion der Stufe (b) vorteilhaft in Gegenwart eines Überschusses des Amins in einem niederen Alkanol als inertem Lösungsmittel durchgeführt wird.

6. Arzneimittelzubereitung mit insbesondere $\beta$-adrenergische Rezeptoren hemmenden und antiarrhythmischen Wirkungen, dadurch gekennzeichnet, daß sie als Wirkstoff eine Verbindung, wie sie in Anspruch 1 definiert wurde, enthält.

7. Arzneimittelzubereitung nach Anspruch 6, dadurch gekennzeichnet, daß sie in einer für die orale, parenterale oder rektale Verabreichung geeigneten Form vorliegt und der Wirkstoff mit therapeutisch unbedenklichen Trägern oder Hilfsstoffen verbunden ist.

8. Arzneimittelzubereitung nach Anspruch 6, dadurch gekennzeichnet, daß sie in Form von Dosierungseinheiten vorliegt, die jeweils 0,005 bis 0,100 g Wirkstoff enthalten.

**Claims**

1. Derivatives of 1-aminopropan-2-ol of the general formulae:

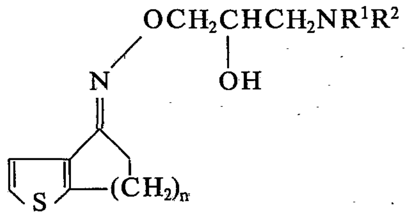

E-isomer E (I)

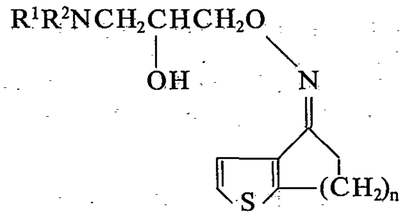

Z-isomer Z (II)

wherein $R^1$ and $R^2$ represent, independently from each other, a hydrogen atom, a straight-chained or branched alkyl radical having from 1 to 10 carbon atoms; a mono- or polycyclic cycloalkyl radical having from 1 to 14 carbon atoms optionally mono- or polysubstituted by an alkyl radical having from 1 to 4 carbon atoms; a straight-chained or branched alkenyl radical having from 3 to 7 carbon atoms; a straight-chained or branched alkenyl radical having from 3 to 7 carbon atoms; a phenyl radical or a phenylalkyl radical having from 1 to 4 carbon atoms in the alkyl moiety, the aromatic nuclei being optionally mono- or polysubstituted with a halogen atom, a hydroxyl group, a ($C_1$ to $C_4$) alkyl or a ($C_1$ to $C_4$) alkoxy radical; a pyridyl radical or a pyridylalkyl radical having from 1 to 4 carbon atoms in the alkyl moiety; a ($C_1$ to $C_4$) aminoalkyl radical, optionally mono- or di-substituted on the nitrogen atom by a ($C_1$ to $C_4$) alkyl radical; or $R^1$ and $R^2$, together with the nitrogen atom to which they are attached, form a heterocyclic radical containing 5 to 7 ring members and optionally containing another heteroatom selected from oxygen, sulphur and nitrogen, the latter being optionally substituted by a $C_1-C_6$ alkyl radical, a phenyl radical, a phenylalkyl radical, the aromatic nucleus of which is optionally mono- or polysubstituted by a halogen atom, a hydroxy group, a ($C_1$ to $C_4$) alkyl, or a ($C_1$ to $C_4$) alkoxy radical, and n is an integer from 1 to 3; and the addition salts thereof with pharmaceutically acceptable inorganic or organic acids.

2. Derivatives according to claim 1 having the general formulae I and II, wherein $R^1$ and $R^2$, which represent, independently from each other, a hydrogen atom; a straight-chained or branched alkyl radical having from 1 to 8 carbon atoms; a monocyclic cycloalkyl radical having from 1 to 12 carbon atoms or an adamantyl radical; an $\alpha,\alpha$-di($C_{1-4}$)alkyl propynyl radical; a phenyl($C_{1-4}$)alkyl radical optionally mono- or poly-substituted on the phenyl nucleus by a $C_1$ to $C_4$ alkoxy radical; a pyridyl($C_{1-4}$)alkyl radical; or a di($C_{1-4}$)alkylamino($C_{1-4}$)alkyl radical;
or $R^1$ and $R^2$, together with the nitrogen atom to which they are attached, form a pyrrolidino, morpholino piperidino oı piperazino radical, the latter optionally being substituted on the other nitrogen atom by a ($C_1$ to $C_4$) alkyl radical or by a benzyl radical; and n is an integer from 1 to 3; and the addition salts thereof with pharmaceutically-acceptable inorganic or organic acids.

3. Process for the preparation of derivatives of 1-aminopropan-2-ol according to claim 1, which comprises

a) condensing oximes of the following formulae (III) or (IV):

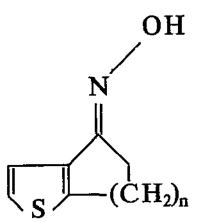

(III)

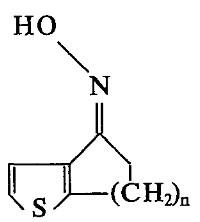

(IV)

with an epihalohydrin in an inert solvent, in the presence of a base and at a temperature of from 10° C to the boiling point of the solvent, to give compounds of the formulae:

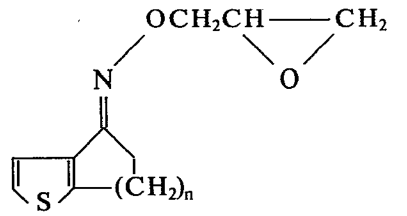

(V)

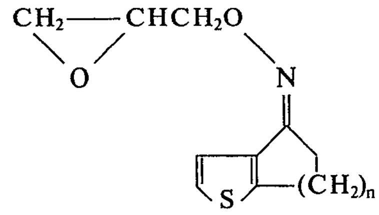

(VI)

in which n has the meaning previously given, and

b) condensing the compounds obtained of general formulae (V) and (VI) with an amine of the formula $HNR^1R^2$, in which $R^1$ and $R^2$ are as defined above, in an inert solvent and at a temperature of form 10°C to the boiling point of the solvent, to give respectively the derivatives of general formulae (I) or (II).

4. Process according to claim 3, wherein the condensation reaction in stage a) is carried out using epichlorohydrin in an inert solvent selected from dimethylformamide, methanol and ethanol in the presence of an alkali metal carbonate as the base.

5. Process according to claim 3, wherein the condensation reaction in stage b) is advantageously carried out in the presence of an excess of the amine, using a lower alkanol as the inert solvent.

6. Medicament having notably $\beta$-adrenergic receptor inhibiting and antiarrhythmic activities, comprising as active principle a compound according to claim 1.

7. Medicament according to claim 6, in a form suitable for oral, parenteral or rectal administration, the active principle being associated with therapeutically acceptable vehicles or excipients.

8. Medicament according to claim 6 in the form of a dosage unit containing 0.005 to 0.100 g of active principle.